(19) Europäisches Patentamt
European Patent Office
Office européen des brevets

(11) Veröffentlichungsnummer: **0 474 057 B1**

(12) **EUROPÄISCHE PATENTSCHRIFT**

(45) Veröffentlichungstag der Patentschrift: **08.11.95**

(51) Int. Cl.$^{6}$: **C07D 213/61**

(21) Anmeldenummer: **91114128.1**

(22) Anmeldetag: **23.08.91**

Die Akte enthält technische Angaben, die nach dem Eingang der Anmeldung eingereicht wurden und die nicht in dieser Patentschrift enthalten sind.

# (54) Verfahren zur Herstellung von N-(2-Chlor-pyridin-5-yl-methyl)-ethylendiamin.

(30) Priorität: **05.09.90 DE 4028047**

(43) Veröffentlichungstag der Anmeldung:
**11.03.92 Patentblatt 92/11**

(45) Bekanntmachung des Hinweises auf die Patenterteilung:
**08.11.95 Patentblatt 95/45**

(84) Benannte Vertragsstaaten:
**BE CH DE ES FR GB IT LI NL**

(56) Entgegenhaltungen:
**DE-A- 3 603 100**

**CHEMICAL ABSTRACTS, Band 87, Nr. 3, 18. Juli 1977, Columbus, Ohio, USA SUMITOMO-CHEM. "N-substituted aminoaceto- nitriles as fungicides" Seite 165, Spalte 2, Zusammenfassung-Nr. 17 298x**

**CHEMICAL ABSTRACTS, Band 94, Nr. 13, 30. MUrz 1981, Columbus, Ohio, USA SHOWA-DENKO "Aminonitriles" Seite 692, Spalte 1, Zusammenfassung-Nr. 102 862r**

**Methoden der Organischen Chemie (Houben-Weyl), B. XI/1, S. 554-67**

**Methoden der Organischen Chemie (Houben Weyl), B. IV/1c, S. 132-3**

**Compendium of Organic Synthetic Methods, Wiley Interscience, S. 262**

(73) Patentinhaber: **BAYER AG**

**D-51368 Leverkusen (DE)**

(72) Erfinder: **Diehr, Hans-Joachim, Dr.**
**Höhe 35**
**W-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein neues Verfahren zur Herstellung von N-(2-Chlor-pyridin-5-yl-methyl)-ethylendiamin und N-(2-Chlor-pyridin-5-yl-methyl)-aminoacetonitril als neues Zwischenprodukt hierfür.

Es ist bekannt, daß man N-(2-Chlor-pyridin-5-yl-methyl)-ethylendiamin,ein Zwischenprodukt für Insektizide erhält, wenn man 2-Chlor-5-chlormethyl-pyridin mit Ethylendiamin in Acetonitril umsetzt (vgl. EP-A 163855, Beispiel 1).

Diese Umsetzung verläuft jedoch nicht ausreichend selektiv, was zu einer Minderung der Ausbeute am gewünschten Produkt führt.

Als allgemeine Methode zur Herstellung von Ethylendiamin-Derivaten ist die katalytische Hydrierung von entsprechenden Aminoacetonitril-Derivaten bekannt. An heteroaromatischen Komponenten befindliche Halogen-Substituenten - und allgemein auch Blausäure - werden hierbei jedoch leicht abgespalten; insgesamt ist der selektive Verlauf der Hydrierung - soweit im Einzelfall überhaupt befriedigend erreichbar - meist empfindlich von der Einhaltung bestimmter Reaktionsbedingungen (gegebenenfalls hoher Druck/hohe Temperatur/spezieller kostenaufwendiger Katalysator etc.) abhängig (vgl. Houben-Weyl, Methoden der organischen Chemie, Band IV/1c (1980), S. 127-132; loc. cit. Band XI/1 (1957), S. 554-567).

Es wurde nun ein Verfahren zur Herstellung von N-(2-Chlor-pyridin-5-yl-methyl)-ethylendiamin der Formel (I)

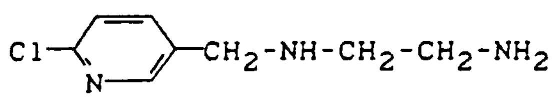

(I)

gefunden, welches dadurch gekennzeichnet ist, daß man N-(2-Chlor-pyridin-5-yl-methyl)-aminoacetonitril der Formel (II)

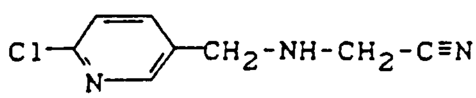

(II)

mit Wasserstoff in Gegenwart von Ammoniak und in Gegenwart eines Hydrierkatalysators der Reihe Raney-Nickel, Platin, Rhodium und Palladium sowie in Gegenwart eines gegenüber Wasserstoff inerten Lösungsmittels der Reihe Alkohole, Ether oder Ester bei Temperaturen zwischen 0 °C und 100 °C und einem Druck zwischen 1 bar und 100 bar umsetzt.

Überraschenderweise kann nach dem erfindungsgemäßen Verfahren N-(2-Chlor-pyridin-5-yl-methyl)-ethylendiamin auf technisch einfache Weise in hoher Ausbeute und in sehr guter Qualität erhalten werden, d.h. die oben beschriebenen Nachteile treten nicht auf.

Durch die Bereitstellung des als Ausgangsverbindung eingesetzten N-(2-Chlor-pyridin-5-yl-methyl)-aminoacetonitrils der Formel (II) wird ein neuer erfinderischer Weg zum Erhalt des bekannten N-(2-Chlor-pyridin-5-yl-methyl)-ethylendiamins(I) eröffnet.

Das erfindungsgemäße Verfahren stellt somit eine wertvolle Bereicherung des Standes der Technik dar.

Der Reaktionsablauf beim erfindungsgemäßen Verfahren kann durch das folgende Formelschema wiedergegeben werden:

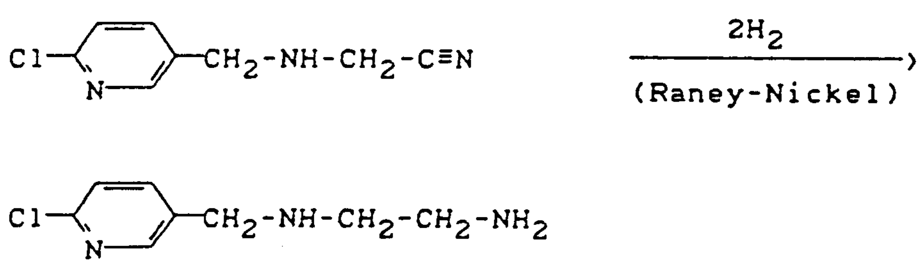


Das beim erfindungsgemäßen Verfahren als Ausgangsstoff zu verwendende N-(2-Chlor-pyridin-5-yl-methyl)-aminoacetonitril ist noch nicht aus der Literatur bekannt und ist ebenfalls Gegenstand der vorliegenden

Erfindung.

Man erhält das neue N-(2-Chlor-pyridin-5-yl-methyl)-aminoacetonitril der Formel (II)

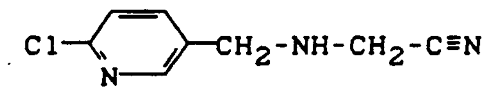

(II)

wenn man 2-Chlor-5-aminomethyl-pyridin der Formel (III)

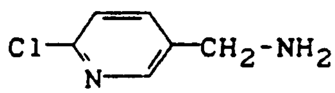

(III)

mit Formaldehyd in Gegenwart von Wasser und gegebenenfalls in Gegenwart eines Reaktionshilfsmittels, wie z.B. Natriumhydrogensulfit, und dann mit einem Cyanid der Reihe Blausäure, Natriumcyanid oder Kaliumcyanid bei Temperaturen zwischen 0 °C und 50 °C umsetzt.

Ferner kann die Verbindung der Formel (II) aus 2-Chlor-5-chlormethyl-pyridin und Aminoacetonitril in Gegenwart von Basen erhalten werden.

Das als Ausgangsstoff benötigte 2-Chlor-5-aminomethylpyridin der Formel (III) ist bereits bekannt (vgl. EP-A 302389).

Das erfindungsgemäße Verfahren zur Herstellung von N-(2-Chlor-pyridin-5-yl-methyl)-ethylendiamin wird in Gegenwart eines Verdünnungsmittels durchgeführt. Es kommen dabei praktisch alle üblichen Lösungsmittel, soweit sie gegenüber Wasserstoff inert sind, in Betracht. Vorzugsweise werden Alkohole, wie Methanol, Ethanol, Propanol, Isopropanol, Butanol, Isobutanol, sec-Butanol und tert-Butanol, insbesondere Ethanol, Ether, wie Diisopropylether, Diisobutylether, Glycoldimethylether, Diglycoldimethylether, Tetrahydrofuran oder Dioxan, oder Ester, wie Essigsäuremethylester oder Essigsäureethylester als Lösungsmittel eingesetzt.

Das erfindungsgemäße Verfahren wird in Gegenwart eines Katalysators durchgeführt. Es kommen hierbei die üblichen, bei katalytischen Hydrierungen zu verwendenden Katalysatoren in Betracht. Als Beispiele hierfür seien Raney-Nickel, Platin, Rhodium und Palladium - letztere gegebenenfalls auf geeigneten Trägermaterialien, wie (Aktiv-)Kohle oder Bariumsulfat - genannt. Vorzugsweise wird Raney-Nickel als Katalysator eingesetzt.

Die Reaktionstemperaturen können beim erfindungsgemäßen Verfahren in einem größeren Bereich variiert werden. Im allgemeinen arbeitet man bei Temperaturen zwischen 0 °C und 100 °C, vorzugsweise bei Temperaturen zwischen 10 °C und 50 °C, insbesondere zwischen 15 °C und 30 °C.

Das erfindungsgemäße Verfahren wird im allgemeinen im Druckbereich zwischen 1 bar und 100 bar, vorzugsweise zwischen 2 bar und 50 bar, insbesondere zwischen 5 bar und 20 bar, durchgeführt.

Zur Durchführung des erfindungsgemäßen Verfahrens setzt man auf 1 Mol N-(2-Chlor-pyridin-5-yl-methyl)-aminoacetonitril der Formel (II) im allgemeinen zwischen 1 und 50 Mol, vorzugsweise zwischen 2 und 20 Mol Ammoniak, zwischen 10 und 200 g, vorzugsweise zwischen 20 und 100 g Katalysator und Wasserstoff bis zum Ende der Aufnahme ein.

Das erfindungsgemäße Verfahren wird unter den bei katalytischen Hydrierungen üblichen Bedingungen durchgeführt. In einer bevorzugten Ausführungsform wird die Ausgangsverbindung der Formel (II) im Verdünnungsmittel vorgelegt, mit dem Katalysator und dem Ammoniak versetzt und dann im Autoklaven unter Rühren so lange mit Wasserstoff behandelt, bis kein weiterer Verbrauch von Wasserstoff mehr festzustellen ist. Nach dem Ende der Hydrierung wird der Katalysator durch Filtration abgetrennt und vom Filtrat wird das Lösungsmittel unter vermindertem Druck sorgfältig abdestilliert. Der verbleibende Rückstand enthält im wesentlichen das Produkt der Formel (I).

Das nach dem erfindungsgemäßen Verfahren herzustellende N-(2-Chlor-pyridin-5-yl-methyl)-ethylendiamin kann als Zwischenprodukt zur Herstellung von Insektiziden verwendet werden (vgl. EP-A 163855).

Als Reaktionsschema für die weitere Umsetzung zum Erhalt insektizider Wirkstoffe sei beispielhaft aufgeführt:

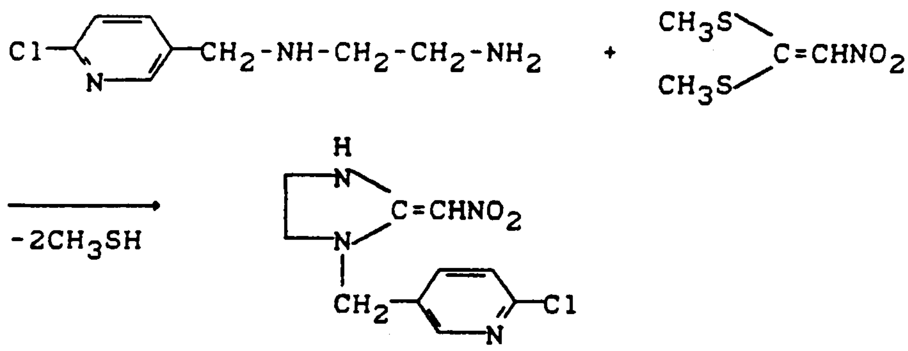


Herstellungsbeispiele:

Beispiel 1

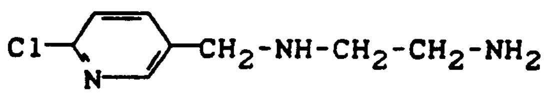

9,1 g (0,05 Mol) N-(2-Chlor-pyridin-5-yl-methyl)-aminoacetonitril werden in 75 ml Ethanol gelöst, mit 3,6 g Raney-Nickel und dann mit 8 ml flüssigem Ammoniak versetzt. Das Gemisch wird in einem Autoklaven bei 20 ° C bis 25 ° C unter einem Wasserstoffdruck von ca. 10 bar 3 Stunden gerührt. Dann wird der Katalysator durch Absaugen abgetrennt und vom Filtrat wird das Lösungsmittel im Wasserstrahlvakuum sorgfältig abdestilliert.

Man erhält 7,8 g (84% der Theorie) N-(2-Chlor-pyridin-5-yl-methyl)-ethylendiamin als gelben, öligen Rückstand. Siedepunkt: 125 ° C - 127 ° C/0,01 mbar.

Ausgangsverbindung der Formel (II):

Beispiel (II-1)

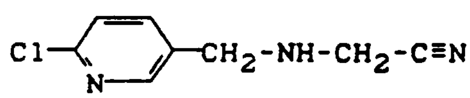

Eine Mischung aus 260 g einer wäßrigen Natriumhydrogensulfit-Lösung (1 Mol $NaHSO_3$) und 100 g einer wäßrigen Formaldehyd-Lösung (1 Mol $H_2CO$) wird mit 144 g (1 Mol) 2-Chlor-5-aminomethyl-pyridin versetzt und das Gemisch wird 105 Minuten bei 25 ° C bis 30 ° C gerührt. Dann wird eine gesättigte wäßrige Lösung von 49 g (1 Mol) Natriumcyanid dazugegeben und das Gemisch wird 15 Stunden bei 20 ° C bis 25 ° C gerührt. Anschließend wird die wäßrige Lösung zweimal mit je 250 ml Methylenchlorid geschüttelt, die vereinigten organischen Extrakte mit Natriumsulfat getrocknet und filtriert. Vom Filtrat wird das Lösungsmittel im Wasserstrahlvakuum sorgfältig abdestilliert.

Man erhält 164 g (90% der Theorie) N-(2-Chlor-pyridin-5-yl-methyl)-aminoacetonitril als gelben, öligen Rückstand vom Brechungsindex $n_D^{22}$ = 1.5520.

**Patentansprüche**

1. Verfahren zur Herstellung von N-(2-Chlor-pyridin-5-yl-methyl)-ethylendiamin der Formel (I)

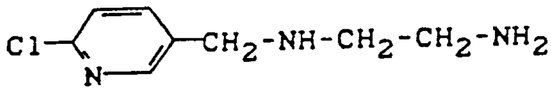

(I)

dadurch gekennzeichnet, daß man N-(2-Chlor-pyridin-5-yl-methyl)-aminoacetonitril der Formel (II)

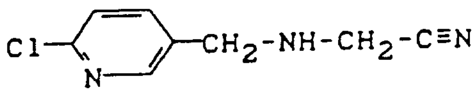

(II)

mit Wasserstoff in Gegenwart von Ammoniak und in Gegenwart eines Hydrierkatalysators der Reihe Raney-Nickel, Platin, Rhodium und Palladium sowie in Gegenwart eines gegenüber Wasserstoff inerten Lösungsmittels der Reihe Alkohole, Ether oder Ester bei Temperaturen zwischen 0 °C und 100 °C und einem Druck zwischen 1 bar und 100 bar umsetzt.

2. Verfahren gemäß Anspruch 1 , dadurch gekennzeichnet, daß in einem Temperaturbereich von 10 °C bis 50 °C gearbeitet wird.

3. Verfahren gemäß Anspruch 1 bis 2, dadurch gekennzeichnet, daß in einem Druckbereich von 2 bar bis 50 bar umgesetzt wird.

4. Verfahren gemäß Anspruch 1 bis 3, dadurch gekennzeichnet, daß man pro Mol N-(2-Chlor-pyridin-5-yl-methyl)-aminoacetonitril der Formel (II) zwischen 1 und 50 Mol Ammoniak, zwischen 10 g und 200 g Katalysator und Wasserstoff bis zum Ende der Aufnahme einsetzt.

5. Verfahren gemäß Anspruch 1 bis 4 , dadurch gekennzeichnet, daß man pro Mol N-(2-Chlor-pyridin-5-yl-methyl)-aminoacetonitril (II) zwischen 2 und 20 Mol Ammoniak, zwischen 20 g und 100 g Katalysator und Wasserstoff bis zum Ende der Aufnahme einsetzt.

6. Verfahren gemäß Anspruch 1 bis 5 , dadurch gekennzeichnet, daß man N-(2-Chlor-pyridin-5-yl-methyl)-aminoacetonitril (II) im Verdünnungsmittel vorlegt, mit dem Katalysator und dem Ammoniak versetzt und dann im Autoklaven unter Rühren so lange behandelt, bis kein weiterer Verbrauch von Wasserstoff mehr festzustellen ist, nach dem Ende der Hydrierung den Katalysator durch Filtration abtrennt und vom Filtrat das Lösungsmittel unter vermindertem Druck abdestilliert.

7. N-(2-Chlor-pyridin-5-yl-methyl)-aminoacetonitril der Formel (II)

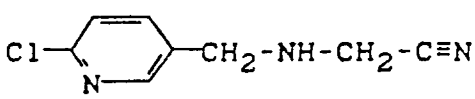

(II) .

8. Verfahren zur Herstellung von N-(2-Chlor-pyridin-5-yl-methyl)-aminoacetonitril der Formel (II)

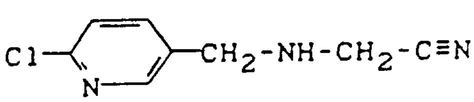

(II)

dadurch gekennzeichnet, daß man entweder

a) 2-Chlor-5-aminomethyl-pyridin der Formel (III)

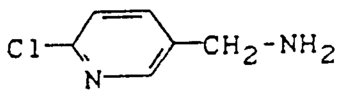

(III)

mit Formaldehyd in Gegenwart von Wasser und gegebenenfalls in Gegenwart eines Reaktionshilfsmittels mit einem Cyanid der Reihe Blausäure, Natriumcyanid oder Kaliumcyanid bei Temperaturen zwischen 0 ° C und 50 ° C umsetzt

oder daß man

b) 2-Chlor-5-chlormethyl-pyridin mit Aminoacetonitril in Gegenwart von Basen umsetzt.

**Claims**

**1.** Process for the preparation of N-(2-chloro-pyridin-5-yl-methyl)-ethylenediamine of the formula (I)

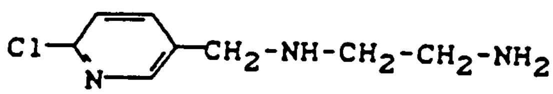

(I),

characterised in that N-(2-chloro-pyridin-5-yl-methyl)-aminoacetonitrile of the formula (II)

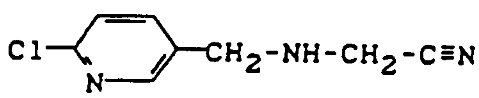

(II)

is reacted with hydrogen in the presence of ammonia and in the presence of a hydrogenation catalyst from the series comprising Raney nickel, platinum, rhodium and palladium and in the presence of a solvent which is inert to hydrogen, from the series comprising alcohols, ethers or esters, at temperatures between 0 ° C and 100 ° C and a pressure between 1 bar and 100 bar.

**2.** Process according to Claim 1, characterised in that the process is carried out in a temperature range from 10 ° C to 50 ° C.

**3.** Process according to Claim 1 to 2, characterised in that the reaction is carried out in a pressure range from 2 bar to 50 bar.

**4.** Process according to Claim 1 to 3, characterised in that between 1 and 50 mol of ammonia, between 10 g and 200 g of catalyst and hydrogen until it is no longer taken up are employed per mole of N-(2-chloro-pyridin-5-yl-methyl)-aminoacetonitrile of the formula (II).

**5.** Process according to Claim 1 to 4, characterised in that between 2 and 20 mol of ammonia, between 20 g and 100 g of catalyst and hydrogen until it is no longer taken up are employed per mole of N-(2-chloro-pyridin-5-yl-methyl)-aminoacetonitrile of the formula (II).

**6.** Process according to Claim 1 to 5, characterised in that N-(2-chloro-pyridin-5-yl-methyl)-aminoacetonitrile (II) is initially introduced in the diluent, the catalyst and ammonia are added, and the mixture is then treated with stirring in an autoclave until further consumption of hydrogen can no longer be detected, and, when the hydrogenation has ended, the catalyst is separated off by filtration and the solvent is removed from the filtrate by distillation under reduced pressure.

**7.** N-(2-Chloro-pyridin-5-yl-methyl)-aminoacetonitrile of the formula (II)

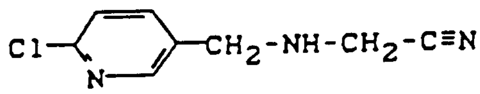

(II).

**8.** Process for the preparation of N-(2-chloro-pyridin-5-yl-methyl)-aminoacetonitrile of the formula (II)

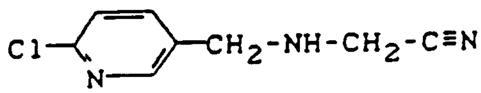

(II),

characterised in that either

a) 2-chloro-5-aminomethyl-pyridine of the formula (III)

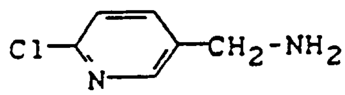

(III)

is reacted with formaldehyde in the presence of water and if appropriate in the presence of a reaction auxiliary with a cyanide from the series comprising hydrocyanic acid, sodium cyanide or potassium cyanide at temperatures between 0 ° C and 50 ° C

or in that

b) 2-chloro-5-chloromethyl-pyridine is reacted with aminoacetonitrile in the presence of bases.

## Revendications

**1.** Procédé de production de N-(2-chloropyridine-5-ylméthyl)-éthylènediamine de formule (I)

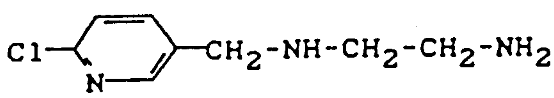

(I)

caractérisé en ce qu'on fait réagir à des températures comprises entre 0 ° C et 100 ° C et sous pression comprise comprise entre 1 bar et 100 bars le N-(2-chloropyridine-5-yl-méthyl)-aminoacétonitrile de formule (II)

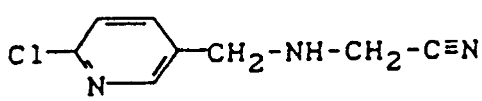

(II)

avec de l'hydrogène en présence d'ammoniac et en présence d'un catalyseur d'hydrogénation de la série du nickel de Raney, du platine, du rhodium et du palladium ainsi qu'en présence d'un solvant inerte vis-à-vis de l'hydrogène, de la série des alcools, des éthers ou des esters.

**2.** Procédé suivant la revendication 1, caractérisé en ce qu'on opère dans une plage de températures de 10 ° C à 50 ° C.

**3.** Procédé suivant les revendications 1 et 2, caractérisé en ce qu'on conduit la réaction dans une plage de pression de 2 bars à 50 bars.

4. Procédé suivant les revendications 1 à 3, caractérisé en ce qu'on utilise par mole de N-(2-chloropyridine-5-ylméthyl)-aminoacétonitrile de formule (II) entre 1 et 50 moles d'ammoniac, entre 10 g et 200 g de catalyseur et de l'hydrogène jusqu'à la fin de l'absorption.

5. Procédé suivant les revendications 1 à 4, caractérisé en ce qu'on utilise, pour chaque mole de N-(2-chloropyridine-5-ylméthyl)-aminoacétonitrile (II), entre 2 et 20 moles d'ammoniac, entre 20 g et 100 g de catalyseur et de l'hydrogène jusqu'à la fin de l'absorption.

6. Procédé suivant les revendications 1 à 5, caractérisé en ce qu'on charge tout d'abord le N-(2-chloropyridine-5-ylméthyl)-aminoacétonitrile (II) dans le diluant, on ajoute le catalyseur et l'ammoniac puis on traite le mélange sous agitation en autoclave jusqu'à ce qu'on ne puisse plus déceler d'autre consommation d'hydrogène, on sépare le catalyseur par filtration lorsque l'hydrogénation est terminée et on chasse le solvant du filtrat par distillation sous pression réduite.

7. Le N-(2-chloropyridine-5-ylméthyl)-aminoacétonitrile de formule (II)

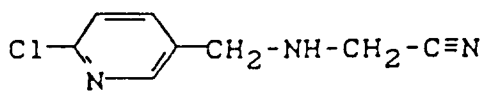

(II) .

8. Procédé de production de N-(2-chloropyridine-5-ylméthyl)-aminoacétonitrile de formule (II)

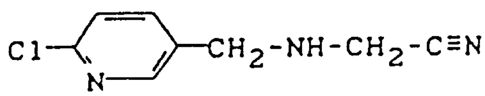

(II)

caractérisé en ce que :

a) on fait réagir à des températures comprises entre 0 ° C et 50 ° C la 2-chloro-5-aminométhylpyridine de formule (III)

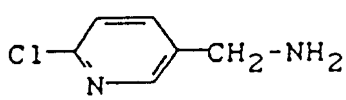

(III)

avec le formaldéhyde en présence d'eau et, le cas échéant, en présence d'une substance auxiliaire de réaction, avec un cyanure de la série acide cyanhydrique, cyanure de sodium ou cyanure de potassium,

ou bien

b) on fait réagir la 2-chloro-5-chlorométhylpyridine avec l'aminoacétonitrile en présence de bases.